# EUROPEAN PATENT SPECIFICATION

(11) **EP 0 850 319 B1**
(45) Date of publication and mention of the grant of the patent: **24.03.2004**
(21) Application number: 96930710.7
(22) Date of filing: 04.09.1996
(51) Int. Cl.: C12Q 1/68, G01N 33/53, G01N 33/567, C12N 5/00

(54) **METHOD FOR MODULATING CELL APOPTOSIS**
VERFAHREN ZUR MODULATION DER ZELL-APOPTOSE
PROCEDE DE MODULATION DE L'APOPTOSE CELLULAIRE

(30) Priority: 12.09.1995 GB 9518611; 10.10.1995 US 541905
(43) Date of publication of application: 01.07.1998
(60) Divisional application: 04000411.1
(73) Proprietor: LUDWIG INSTITUTE FOR CANCER RESEARCH, New York, New York 10105 (US)
(72) Inventor: VAN DAMME, Jo, B-1040 Brussels (BE); PROOST, Paul, B-3001 Heverlee (BE); HOUSSIAU, Frederic, B-1020 Brussels (BE); RENAULD, Jean-Christophe, B-1030 Brussels (BE)
(74) Representative: Harrison Goddard Foote
(86) International application number: PCT/US1996/014239
(87) International publication number: WO 1997/010361

(56) References cited:
- M. MILLER ET AL.: "The human cytokine I-309 s a monocyte chemoattractant." PROCEEDINGS OF THE NATIONAL ACADEMY OF SCIENCES OF THE U.S.A., vol. 89, no. 7, 1 April 1992, WASHINGTON, DC, USA, pages 2950-2954, XP002066560
- J. VAN SNICK ET AL.: "I-309 /T cell activation gene-3 chemokine protects murine T cell lymphomas against dexamethasone-induced apoptosis." THE JOURNAL OF IMMUNOLOGY, vol. 157, no. 6, 15 September 1996, BALTIMORE, MD, USA, pages 2570-2576, XP002066561
- J. IMMUNOL., 1994, Vol. 153, LANING et al., "Inhibition of In Vivo Tumor Growth by the B Chemokine, TCA3", pages 4625-4635.
- J. IMMUNOL., 1994, Vol. 153, LUO et al., "Biologic Activities of the Murine B-Chemokine TCA3", pages 4616-4624.
- Ruckes T. et al: 'Autocrine antiapoptotic stimulation of cultured adult T-cell leukemia cells by overexpression of the chemokine I-309', Blood (2001), Vol. 98, Nb. 4, pages 1150-1159
- Lee S. et al: 'CCR8 on Human Thymocytes Functiones as a Human Immunodeficiency Virus Type 1 Coreceptor', J. of Virology (2000), Vol 74, Nb. 15, pages 6946-6952
- Goya et al: 'Identification of CCR8 as the Specific Receptor for the Human beta Chemokine I-309:...', J. Immunology (1998), Vol 160, pages 1975-1981
- Ortiz A. et al: CURRENT DRUG TARGETS - IMMUNE, ENDOCRINE & METABIC DISORDERS (2002), Vol. 2, pages 181-192
- Rana A. et al: APOPTOSIS (2001) Vol. 6, pages 83-102
- Dispersyn G.D.; Borgers M.: NEWS PHYSIOL. SCI., (2001), Vol. 16, pages 41-47
- Balig R.R.: CARDIOVASCULAR DESEASE, (2001), Vol. 19, nb. 1, pages 91-112
- Krijnen P.A. et al: J. CLIN. PATHOL., (2002), Vol. 55, pages 801-811
- Mattson M.P. et al: APOPTOSIS (2001), vol. 6, pages 69-81

## Description

### FIELD OF THE INVENTION

The invention relates to the use of a mammalian anti-apoptotic factor which is a chemokine. More particularly the invention concerns the use of the β chemokine I-309 as anti-apoptotic factor.

### BACKGROUND OF THE INVENTION

Chemokines are a subset of cytokines. Cytokines are regulatory proteins secreted by white blood cells and by a variety of other cells in the body. The pleiotropic actions of cytokines include numerous effects on cells in the immune system and the modulation of inflammatory responses ("The Cytokine Handbook", 2nd edition, 1994, ed. A. Thompson, Academic Press, p. 4). Chemokines are a superfamily of proinflammatory cytokines. The chemokine family consists of two branches. In the α chemokines, the first two cysteines are separated by a single amino acid residue (C-X-C) (e.g., IL-8 and platelet factor-4) whereas, in. β chemokines such as TCA-3, the first two cysteines are adjacent (C-C) (Laning et al., 1994, *J. Immunol.* **153**: 4625-4635. Molecules in this family share at least 25% amino acid homology, have similar structures, and bind to seven-transmembrane-spanning receptors of the rhodopsin superfamily. The genes encoding the α chemokines are located on human chromosome 4 and the genes encoding the β chemokines are located on human chromosome 17.

Of the β chemokines, the murine protein TCA-3 was originally described by Burd *et al.* (1987, *J. Immunol.* 139: 3126-3131). Its cDNA was cloned from a T cell helper clone library using a strategy aimed at identifying genes whose expression is induced upon stimulation. Two years later, Brown *et al.* (1989, *J. Immunol.* 142: 679-687) similarly identified a cDNA called P500, which is identical to TCA-3, except for a 99 nucleotide insertion that was generated by alternative splicing in 8% of the cDNA clones that they analyzed. In this paper, TCA-3/P500 was also referred to as SISe (Small Inducible Secreted protein). The sequence of the human I-309 was reported by Miller *et al.* (1989, *J. Immunol.* 143: 2907-2916). Its cDNA was isolated from an IL-2 dependent T cell line using a similar strategy as reported in the preceding papers for TCA-3 and P500. The sequence of a genomic clone was reported later by Miller *et al.* (1990, *J. Immunol.* 145: 2737-2744) and the gene was shown to map to human chromosome 17 as expected for a β chemokine. The genomic structure supports the view that TCA-3 is the murine homologue of I-309.

There are three published reports describing the activity of an example of a β chemokine, that is human I-309, and the murine homologue TCA-3. These are cited herein. In the first report Wilson *et al.* (1990, *J. Immunol.* **145**: 2745-2750) have produced recombinant murine TCA-3 in CHO cells. They showed that intradermal injection of this material into mouse footpads results in a rapid swelling response, histologically characterized by a local accumulation of neutrophils.

In the second report Miller *et al.* (1992, *Proc. Natl.* Acad. *Sci. USA* 89: 2950-2954) produced recombinant human I-309 in CHO cells and showed *in vitro* that purified I-309 stimulated migration of human monocytes but not neutrophils. I-309 was also shown to induce a transient increase in cytoplasmic free calcium in monocytes but not in neutrophils or lymphocytes. The chemotactic activity required a 10 nM concentration (approximately 150 ng/ml) to be detectable. Maximal effect was found at 100-300 nM and half-maximal activity at approximately 20 nM.

Laning *et al.* (1994, *J*. *Immunol.* 153: 4625-4635) showed that a mouse tumor expressing TCA-3 lost its tumorigenicity, probably as a result of the anti-tumor immune response following the attraction of neutrophils and macrophages to the tumor site. "Luo *et al* (J. Immunol. (1994) 135, 4616-4624) also report the proinflammatory activity of TCA-3 through chemotaxis of neutrophils and macrophages. In neither Laning *et al* or Luo *et al* is the anti-apoptotic activity of I309 disclosed."

As previously mentioned, the human β chemokine I-309 was first described in 1989 by Miller *et al.* (1989, *J*. *Immunol.* **143**: 2907-2916). The amino acid and cDNA sequences are described therein, along with other structural characteristics, such as a small polypeptide chain including a hydrophobic leader sequence which was thought to be involved in secretion of the corresponding protein. The function of the protein was unknown at that time, but it was suggested that because of the nature of the protein, that is, its relatively small size, secretion by T cells and other certain structural characteristics, it was a cytokine of unknown function. It has now been found that certain *β* cytokines, including P500 and TCA-3, I-309 demonstrate anti-apoptotic activity, when compared with the known anti-apoptotic agent IL-9, taught as a strong anti-apoptotic factor for thymic lymphoma cell lines. When one considers the effective chemotactic concentration of those proteins discussed infra, one concludes they are strong anti-apoptotic agents. Moreover, given the structural similarities between members of the β chemokine group and also the functional similarities, that is to say distinct chemokines have been shown to bind to the same receptor, it is considered that other members of the β chemokine group will also display anti-apoptotic activity. The following references provide details of the β chemokine group and thus highlight the similarity between members of same. T. J. Schall (1991, *Cytokine* 3: 165-183); and Schall (1994, "The Cytokine Handbook", 2nd edition, ed. A. Thompson, Academic Press, pg. 419-460).

"Apoptosis", as used herein, describes one type of cell death. More particularly, it describes programmed cell death where, in the normal course of development, cells die and dismantle in a predictable and orderly fashion. For example, such cells tend to shrink within their membrane and their DNA is broken into.nucleosomes for safe removal by cells of the immune system and in particular white blood cells.

The scientific community is only now recognizing the clinical importance of apoptosis and therefore only now beginning to invest in research that provides a greater understanding of the process and how the process can be advantageously manipulated in order to either promote, or in some instances, prevent cell death.

There are many examples of disorders and pathologies wherein apoptosis is implicated.

Heart attack is typically characterized by cardiac cell death and it therefore follows that factors that mitigate or prevent this cell death could be used to advantage to protect against the consequences of this condition. In addition, such factors could also be used to advantage in chemotherapy-induced cardiac tissue damage, or in stroke-induced tissue damage or even in kidney failure. The use of such factors would be particularly advantageous where an individual has a history of heart attacks or strokes or even belongs to a family having such a history.

Chemotherapy and radiotherapy also induce other forms of tissue damage and therefore it follows that co-administration or sequential administration of chemotherapy or radiotherapy with an anti-apoptotic factor may be used to advantage in order to mitigate or prevent this widespread damage.

In addition, premature and widespread apoptosis has been implicated in much of the damage associated with acquired immune deficiency syndrome (AIDS). It is thought that apoptosis may be a principal cause of death in uninfected T cells in AIDS patients, leading ultimately to the suppression of the patient's immune system. Thus factors which mitigate or prevent this form of cell death may have a role to play in preventing the damage that the AIDS virus causes in uninfected cells.

Cell death also occurs in instances of ischemia and thus the factors of the invention may also have a role to play in mitigating or preventing apoptosis in ischaemic organs.

In addition to uses to mitigate or prevent apoptotic activity, it is also apparent that one of the ramifications of the disclosure is the ability to promote apoptotic activity when desired. For example, the realization that chemokines, and in particular β chemokines such as I-309 and homologues in other species, have a role to play in preventing apoptosis, can be used to advantage in the manufacture of agents that selectively bind to this factor and thus suppress its anti-apoptotic function, thereby promoting cell death. Naturally occurring, artificial or synthetic agents may be used. For example, antibodies, monoclonal or polyclonal, can be made against the factor and then used to bind to same with a view to suppressing or annihilating its anti-apoptotic activity. Thus agents which selectively bind with the anti-apoptotic factor can be used to promote apoptosis and moreover, having regard to the targeted delivery of such agents, can be used selectively to promote apoptosis of diseased tissue.

It may be advantageous to promote apoptosis in order to suppress or reverse tumor development. In this respect, it is of note that anti-apoptotic factors, for example, the product of the bcl-2 gene which encodes a cytoplasmic, protein that protects against apoptosis without inducing proliferation by itself, has been shown to be involved in some types of tumours.

In addition to the above, the disclosure can also be used in drug screening assays. For example, identifying chemokines, especially β chemokines in different parts of the body, either directly or indirectly, can be used to map apoptosis in various parts of the body and thus areas, regions or organs most likely to suffer from cell death can be identified for drug screening purposes. In addition, the efficacy of apoptotic promoting agents and also agents acting as β chemokines, in particular I-309 antagonists, can more accurately be determined by observing the activity of such agents in the presence of the factor of the invention. In addition, the disclosure can also be used to promote cell growth and proliferation and therefore can be used to provide proliferative tissue for drug screening assays.

The full scope of the invention as it is defined in the appended claims will be understood more fully following review of the disclosure which follows:

### BRIEF DESCRIPTION OF THE FiGURES

Figure 1A shows anti apoptotic activity of various cell supernatants.
Figure 1B shows IL-6 content of the supernatants of figure 1A.
Figure 2 shows inhibition by anti IL-6 antibody of the activity of some, but not all fractions of activated CRL8066-MoT supernatants.
Figure 3 shows distinct fractionation of IL-6 and anti-apoptotic activity on phenyl sepharose.
Figure 4A depicts final purification of the anti-apoptotic factor on a Resource S column.
Figure 4B presents results of activity assay of various column fractions.
Figure 4C is an SDS-gel of various fractions.
Figure 5A shows anti apoptotic and chemotactic activities of purified I-309 on BW5147 cells, when dexamethasone is present, while figure 5B shows its activity in human THP1 monocytes.
Figure 6A sets forth data which shows that BW5147 cells proliferated when induced by recombinant I-309 expressed in COS cells, while figure 6B shows proliferation when stimulated by recombinant TCA 3 in the presence of dexamethasone.
Figure 7A sets forth evidence of recombinant I-309's anti-apoptotic activity on BW5147 cells.
Figure 7B presents electrophoretic data from DNA extracted from BW5147 cells, cultured in DEX with or without recombinant I-309.
Figures 8A and 8B present data showing inhibition of I-309 induced proliferation, in the presence of Bortadella pertussis toxin.
Figure 9 shows the anti-apoptotic activity of I-309 on thymic lymphoma cell lines 9T4A2 and NM3T2, following exposure to dexamethasone.
Figure 10 compares the anti-apoptotic effect of various chemokines.

### Detailed Description of Preferred Embodiments

The invention concerns the anti-apoptotic activity of the β chemokine I-309. Briefly, the investigations described herein show that murine thymic lymphomas can be made to maintain significant proliferative activity and/or survival in the presence of the factor I-309. The anti-apoptotic activity of the factor is significant in that half maximal anti-apoptotic activity was obtained as a concentration of only 1.6 ng/ml. This concentration is considerably lower (200 times lower) than the concentration required for monocyte chemotactic activity of this molecule suggesting that the primary function of this molecule may be suppression of cell death.

In the following data specific reference is made to BW5147 murine thymic lymphoma. However it is of note that similar activity has also been obtained for two other lymphoma cell lines, i.e., 9T4A2 and NM3T2.

### Materials and Methods

### Cell cultures, cytokines and other reagents.

Thymic lymphoma cell line BW5147 (Ralph *et al.*, 1973, *J. Immunol.* 110: 1470) was obtained from the American Type Culture Collection (ATCC). NM3T2 cell line was established from a DBA-2 mouse treated with N-Methyl-N-nitrosourea (MNU) and 9T4A2 from a thymic lymphoma that spontaneously arose in an IL-9-transgenic mouse that had lost the IL-9 transgene (Renauld *et al.*, 1994, *Oncogene* 9: 1327). These cells were cultured in Iscove's medium supplemented with 10% fetal calf serum, 1.5 mM L-glutamine, 0.24 mM L-asparagine, 0.55 mM L-arginine and 50 µM 2-mercaptoethanol.

NK cell leukemia YT and T cell leukemia MoT/CRL8066 (Chen *et al.*, 1983, *Nature* **305**: 502-505) were obtained from the ATCC. Mo7E megakaryoblastic leukemia (Avanzi *et al.*, 1988, *Br. J. Haematol.* **69**: 359-366) was received from Dr. L. Pegoraro (University of Perugia). 5B12 and E5 are human T cell clones derived from blood lymphocytes of healthy individuals and growing in the presence of PHA or allogenic feeder cells, PHA, IL-2 and IL-4.

Murine IL-9 was produced by expression in baculovirus and purified by affinity chromatography in our laboratory. Saturating doses (200U/ml) were used in every experiment. Recombinant TCA-3 was purchased from R&D laboratories and Pertussis Toxin was from BIOMOL (Plymouth Meeting, PA). Dexamethasone (DEX) was obtained from Sigma and used at 0.25 µM concentration in all experiments. The rat anti-murine IL-6 receptor, 15A7 (Coulie *et al,* 1990, *Curr. Top. Microbiol. Immunol.* 166: 43-46), and mouse anti-human IL-6, AH64 (Brailly *et al,* 1994, *Clin. Chem.* **40**: 116-123), antibodies were used at 50ug/ml and at 1/100 dilution of ascites fluid, respectively.

The 7TD1 bioassay used to measure IL-6 was performed as described previously (Van Snick *et al.*, 1986, *Proc. Natl. Acad. Sci. USA* **83**: 9679-9683). Briefly, 2 x 10³ 7TD1 cells were seeded in microwells and cultured for 4 days in the presence of serial dilutions of the supernatants. The proliferation was evaluated by colorimetric determination of hexosamindiase levels as described by Landegren *et al.*, 1984, *Cytokine* 3: 165-183.

### Anti-apoptic bioassays

Cell viability was determined using a propidium iodide incorporation assay as described (Lee *et al.*, 1993, *J*. *Immunol.* 151: 5208). Briefly, cells were incubated for 30 minutes with propidium iodide (125 µg/ml) at room temperature before FACS analysis with a FACScan™ flow cytometer (Becton-Dickinson). Under these conditions, dead cells are brightly stained while live cells are not. A minimum of 5000 cells were counted per sample.

To test the influence of cytokines on proliferation in the presence of DEX, BW5147 cells were incubated in triplicate microtiter wells in the presence of DEX at a final 0.25 x 10⁻⁶ molar concentration with or without cytokines, in a 200 µl volume. Cultures were pulsed 2-4 days later for 6 h with 0.5 µCi ³H-thymidine. Alternatively, cell proliferation was measured by an hexosaminidase assay, as described (Uyttenhove *et al.*, 1988, *Proc. Natl. Acad, Sci, USA* **85**: 6934).

### Purification procedures

MoT/CRL8066, a hairy cell leukemia T type, was cultured in RPMI-1640 medium supplemented with 10% heat-inactivated (56°C, 30 min) FCS, 0.55 mM L-arginine, 0.24 mM L-asparagine, and 1.25 mM L-glutamine. For the production of large batches, cells were washed, resuspended in the same medium containing 5% FCS and stimulated at a concentration of 5 x 10⁵ cells/ml with PMA (50ng/ml) (Sigma). After heating for 30 minutes at 57°C in order to inactivate HTLV-II virus, supernatants were buffered with sodium phosphate, pH 7.0, and ammonium sulfate was added to a final concentration of 1.2 M. This preparation was chromatographed on a Phenyl-Sepharose Fast flow column (Pharmacia) at 8 ml/min. After a 500ml wash with 1.2 M ammonium sulfate, active material was recovered by a two-step elution at 3 ml/ml with 1 liter of 0.7 M and 0.5 M ammonium sulfate.

After concentration, the material was transferred into a sodium acetate 50 mM, pH 5.5 buffer containing 0.01% (vol/vol) Tween 20 before cation exchange chromatography on a Sulfo-Propyl sepharos™ Fast flow gel (Pharmacia) at 3 ml/min. After washes with 100 ml of the same buffer and 140 ml of 0.3 M NaCl in the same buffer, elution was performed with a 300 ml linear gradient of NaCl to 1 M, the active material being eluted at 0.5 to 0.75 M NaCl.

The active fractions were concentrated, transferred into 50 mM phosphate buffer, pH 7.0, containing 1 M NaCl and 0.01% (vol/vol) Tween 20 and fractionated on a Superdex™ 70 size-exclusion column (Pharmacia). The active material (eluted with apparent molecular weight of 12-16 kD) was transferred into 100 mM Na₂HPO₄ buffer pH 7.0 containing 1 M Na₂SO₄ and loaded onto a TSK-phenyl column (LKB, Bromma, Sweden) at 0.5 ml/min. After a 5 min wash in the starting buffer, elution was carried out at 0.5 ml/min with a linear gradient of a 1/1 mixture of sodium phosphate (0.1 M, pH 7.0) and ethylene glycol (from 0 to 60% in 30 min), the active material being eluted at 30% of this buffer.

The active fractions were transferred into sodium acetate 50 mM, pH 5.5 buffer containing 0.01 % (vol/vol) Tween 20 before cation exchange chromatography on a Sulfo-Propyl Resource S column (Pharmacia) at 0.8 ml/min. Elution was performed at 0.8 ml/min with a 10 min linear gradient of NaCl to 0.2 M, followed by a 30 min gradient to 0.4 M NaCl, the active material being eluted at 0.3 M NaCl.

The most active fraction was adjusted to contain 0.1 % trifluoroacetic acid and further analyzed by reverse phase chromatography on a Vydack C4 column. The column was developed at 0.8 ml/min with a 40 min linear gradient from 8 to 56% acetonitrile in 0.1 % trifluoroacetic acid. Fractions were collected in Eppendorf tubes containing 5 µl of Tween 20 (1% in water), lyophilized and resuspended in Na₂HPO₄ buffer (50 mM, pH 7.0) before testing the anti-apoptotic activity.

### cDNA cloning and COS cell transfections

The coding sequence of the human I-309 cDNA was amplified by RT-PCR using total RNA extracted from a PHA activated T cell clone (E5). Reverse transcription was performed on 5 µg total RNA with an oligo(dT) primer, cDNA corresponding to 200 ng of total RNA was amplified for 30 cycles by PCR with the following specific primers into which an EcoRI site was inserted: sense 5'-TCCAGGAATTCCCAAGCCAGACCAGAA-3' (SEQ ID NO:1) (from position 19 of the cDNA sequence), antisense 5'-TTGTAGAATTCAAATGTTTAAAGTGCAACA-3' (SEQ ID NO:2) (from position 503 of the cDNA sequence). The amplification product was digested by EcoRI and cloned in the pCDSRα expression vector (Takebe et al, 1988) The nucleotide sequence was determined by the dideoxynucleotide chain termination method using the Taq cycle sequencing system (Amersham) with ³²P-ATP labelled oligonucleotides, and no difference was observed with the published I-309 sequence (Genbank Accession Number M57502) (Miller *et al.,* 1989, *J. Immunol.* **143**: 2907-2916).

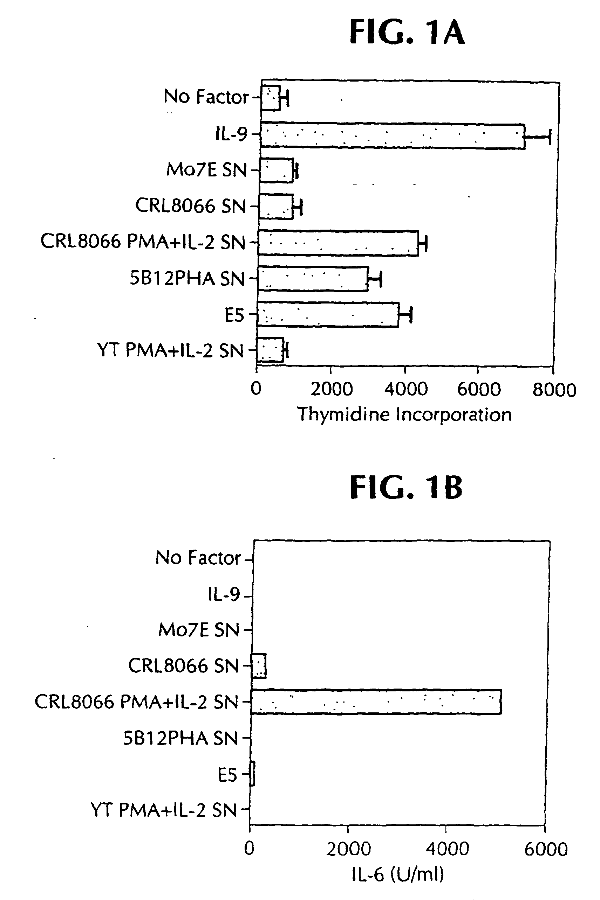

### Anti-apoptotic activity and IL-6 content of various human cell supernatants. (Figure 1)

- A:: For the anti-apoptotic assay, BW5147 cells (10³/well) were incubated for 3 days in the presence of dexamethasone (DEX) (0.25 µM) with or without murine IL-9 (200 U/ml) or 3.3% of supernatant from the Mo7E megakaryoblastic leukemia cell line, MoT/CRL8066 T cell leukemia cell line (stimulated or not with PMA and IL-2 for 3 days) the YT NK cell leukemia stimulated with PMA and IL-2 or 10% of T cell clone supernatants (5B12 and E5). Cell proliferation was evaluated by measuring thymidine incorporation and standard deviations were calculated from triplicate cultures. In the absence of DEX, thymidine incorporation was 117, 947±7,122 cpm.
- B:: The IL-6 content of the supernatants was measured by the 7TD1 bioassay as described. 1 U/ml corresponds, to the concentration required for half-maximal proliferation (approximately 5 pg/ml for human IL-6).

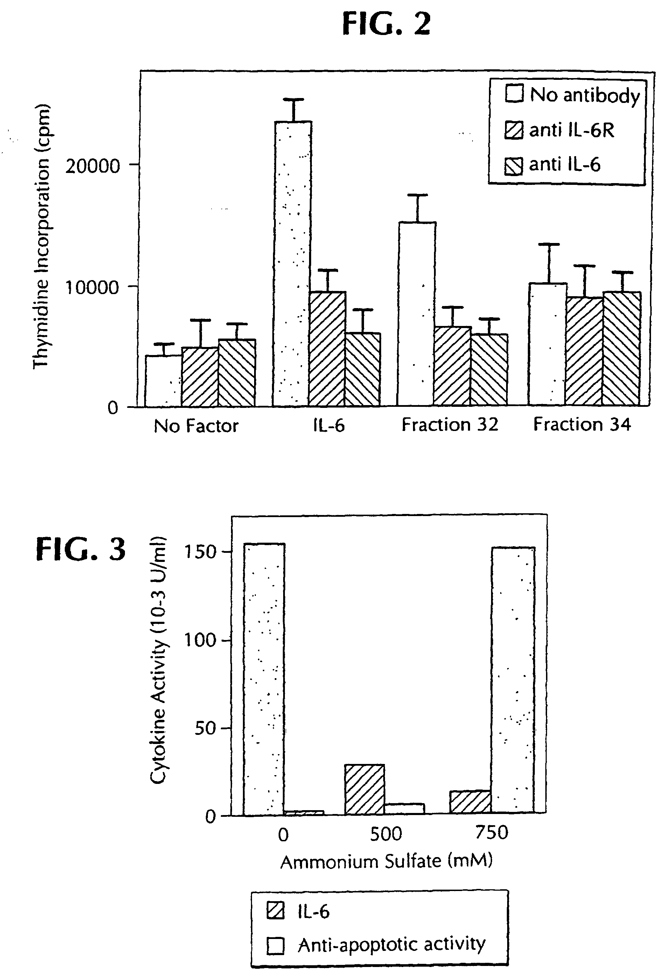

### Inhibition by anti-IL-6 antibody of the activity of some but not all fractions of activated CRL8066-MoT supernatants. (Figure 2)

BW5147 cells (10³/well) were incubated for 3 days in the presence of DEX (0.25 µM) with or without human IL-6 (500 U/ml) or aliquots from elution fractions of the Superose chromatography. The rat anti-murine IL-6 receptor, 15A7, and the mouse anti-human IL-6 antibodies were used at 50 µg/ml and at 1/100 dilution of ascites fluid, respectively. Cell proliferation was evaluated by measuring thymidine incorporation and standard deviations were calculated from triplicate cultures.

### Distinct fractionation of IL-6 and anti-apoptotic activity on phenyl sepharose. (Figure 3)

Supernatants from PMA-stimulated MoT/CRL8066 cells were heated for 30 minutes at 57°C in order to inactivate HTLV-II virus, buffered with sodium phosphate, pH 7.0 before ammonium sulfate was added to a final concentration of 1.2 M and loading on a Phenyl-Sepharose Fast flow column (Pharmacia). After appropriate washing, a three step elution was performed with 0.75, 0.5, and 0.0 M ammonium sulfate and the activity of the fractions was evaluated in the 7TD1 assay for IL-6 and in the BW5147 anti-apototic assay as described in Material and Methods.

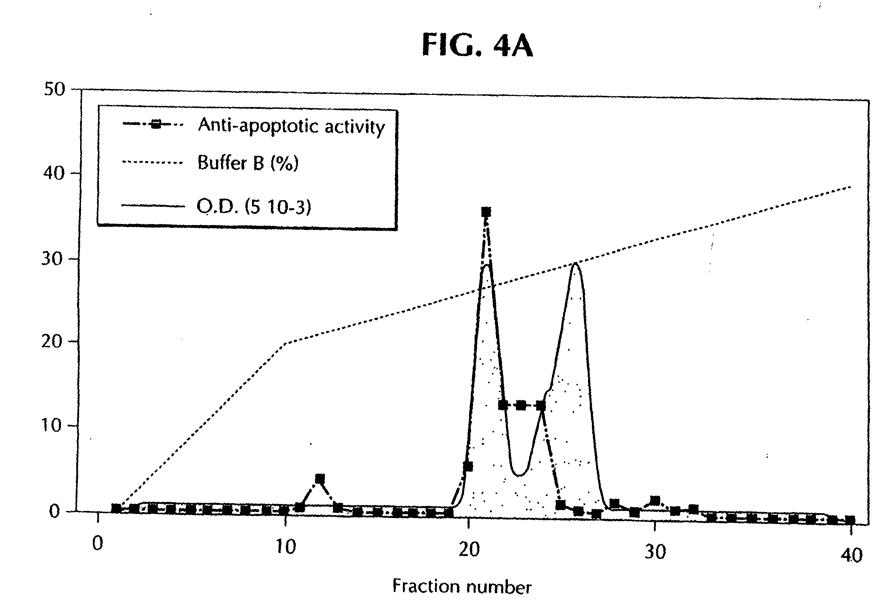

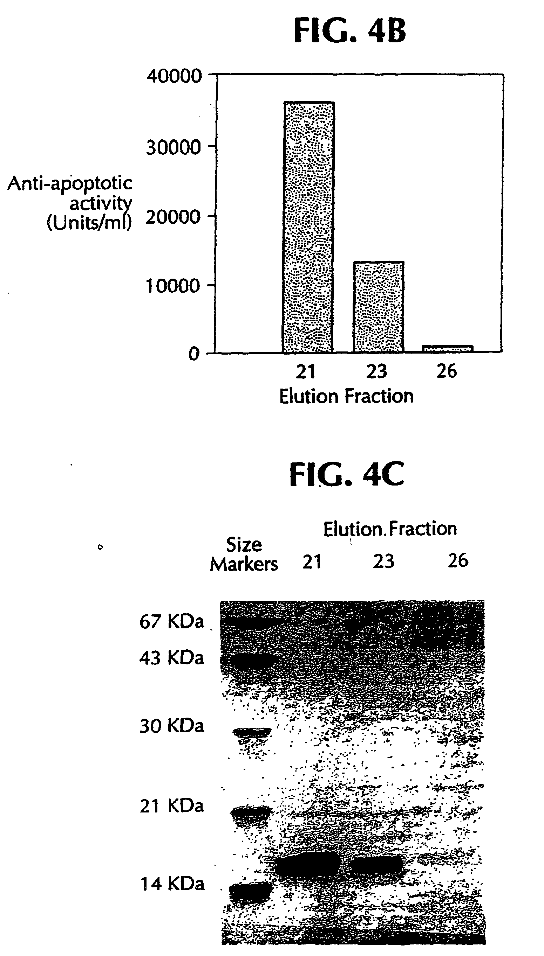

### Final purification of anti-apoptotic factor on Resource S column and SDS PAGE gel. (Figure 4)

- A:: After three successive steps of separation on a phenyl-sepharose gel, Sulfo-Propyl column and Superdex size exclusion column, the active fractions were fractionated by cation-exchange chromatography on a Sulfo-Propyl Resource S column. Elution was performed by a NaCl gradient as shown by the dotted line (Buffer, B = 1M NaCl). The optical density (280 nm) of the eluted material (strippled area) and the anti-apoptotic activity (closed squares) are shown. 1 U/ml of anti-apoptotic activity is arbitrarily defined as the concentration giving half-maximal protection against DEX in the BW5147 bioassay described in Materials and Methods.
- B:: As fractions were removed from the Resource S cation exchange column, they were tested for anti-apoptotic activity in the anti-apoptotic assay described supra. The results, presented in figure 4B, show that elution fraction 21 was, in fact, the fraction containing the apoptotic activity. Several of the fractions were subjected to standard SDS-PAGE analysis, and the results are shown in figure 4C.

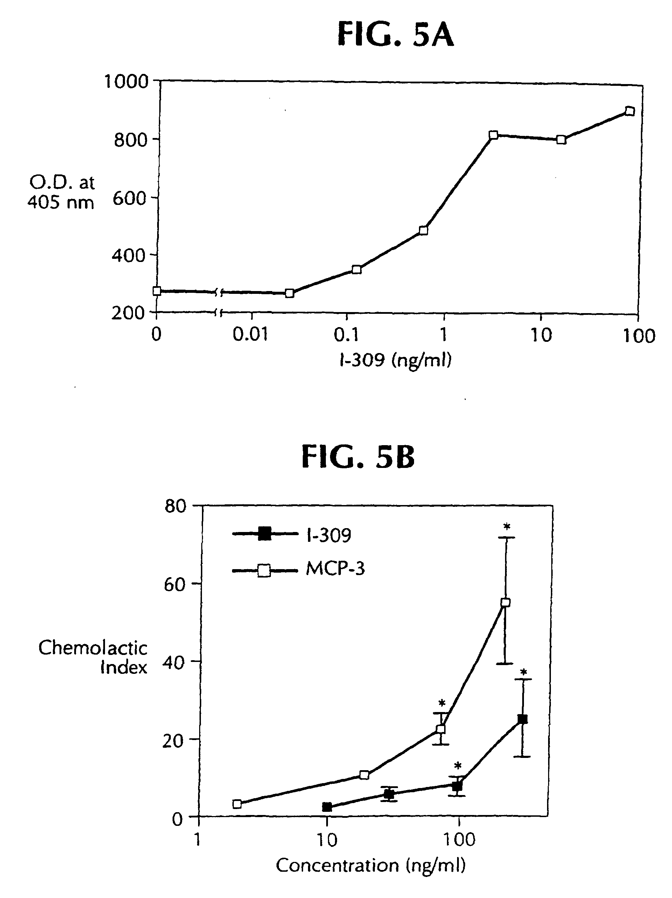

### Anti-apoptotic and chemotactic activities of purified I-309. (Figure 5)

- A:: BW5147 cells (10³/well) were incubated for 3 days in the presence of DEX (0.25 µM) with or without serial dilutions from fraction 21 of the Resource S chromatography shown in Figure 4. Cell proliferation was estimated by a colorimetric hexosaminidase assay. I-309 concentrations were deduced from the micro-sequencing analysis.
- B:: The same method was used in a chemotactic assay using the human THP1 monocytic cell line (closed symbols). Results are indicated as chemotactic indices. Purified human MCP-3 was used as a positive control for this assay (open symbols).

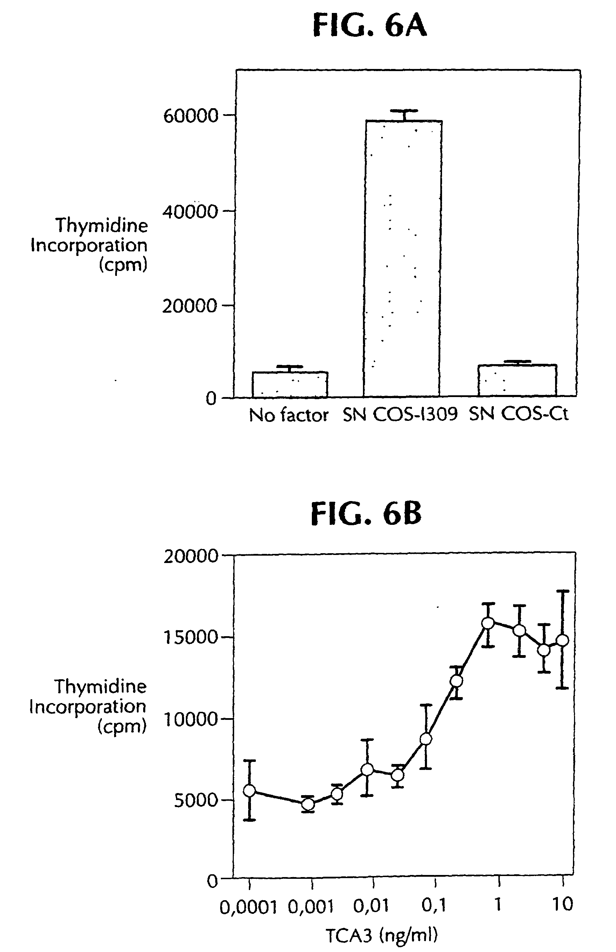

### Proliferation of BW5147 cells induced by recombinant I-309 expressed in COS cells and by recombinant TCA-3 in the presence of dexamethasone. (Figure 6)

- A:: BW5147 cells (10³/well) were incubated for 3 days in the presence of DEX (0.25 µM) with or without a 1/15 dilution of supernatant from COS cells transfected with the I-309 cDNA or a control plasmid. Cell proliferation was evaluated by measuring thymidine incorporation and standard deviations were calculated from triplicate cultures.
- B:: The anti-apoptotic activity of serial dilutions of recombinant murine TCA-3 was measured as described in A.

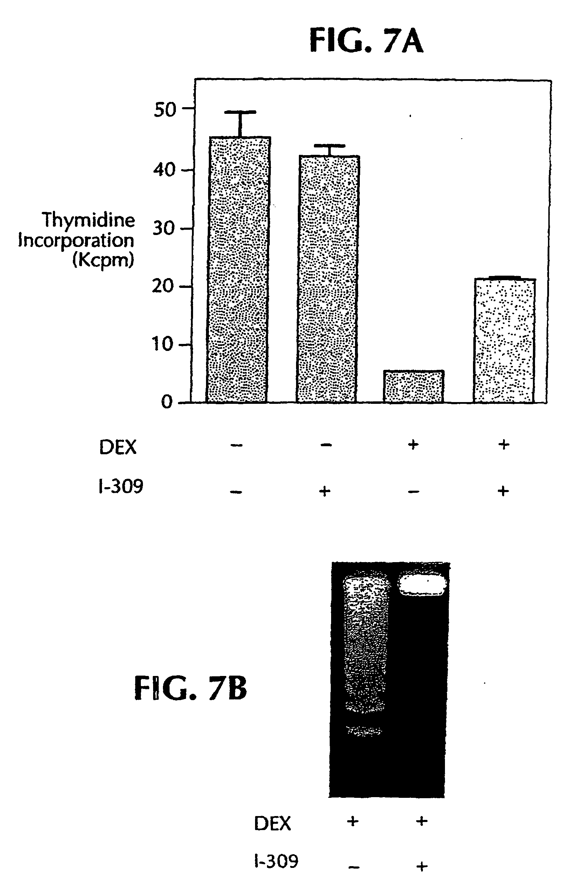

Further work was then carried out using recombinant I-309 on BW5147 cells. In these experiments, the BW 5147 cells (again, 10³/well) , were incubated for 3 days in the presence of 0.25 mM DEX, either with or without purified recombinant I-309 (50 ng/ml). By measuring thymidine incorporation, cell proliferation was evaluated. Triplicate cultures were used to develop the data and to calculate standard deviations. The data are presented in figure 7A. In figure 7B, the results of electrophoresis of DNA which was extracted from BW5147 cells which had been cultured for 24 hours in the presence of DEX with or without 400 ng/ml of recombinant I-309 are shown.

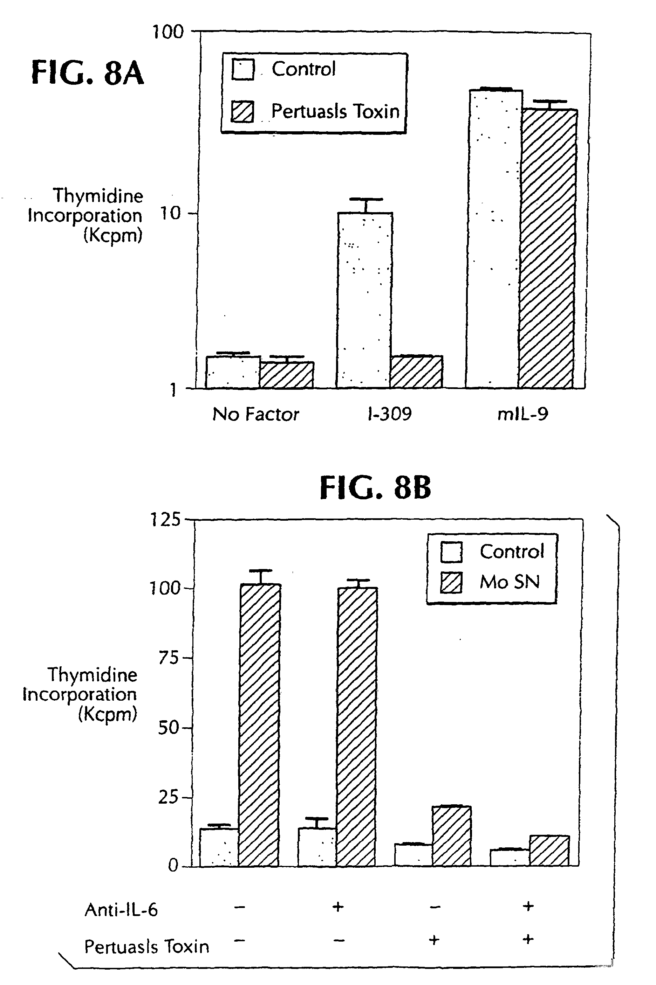

### Inhibition by Pertussis toxin of I-309-induced proliferation of BW514 7 cells in the presence of dexamethasone. (Figure 8)

BW5147 cells (10³/well) were incubated for 3 days in the presence of DEX (0.25 µM) with or without a 1/15 dilution of supernatant from COS cells transfected with the I-309 cDNA or a saturating concentration of mIL-9 (200U/ml). Cell proliferation was evaluated by measuring thymidine incorporation and standard deviations were calculated from triplicate cultures.

In a similar experiment, DEX, at 0.25 mM, with or without 5% activated Mo cell supernatant and with or without B. pertussis toxin (2.5 ng/ml), or anti human IL-6 mAb AH65 (ascites fluid, 0.5%), were added. Thymidine incorporation is shown in figure 8B.

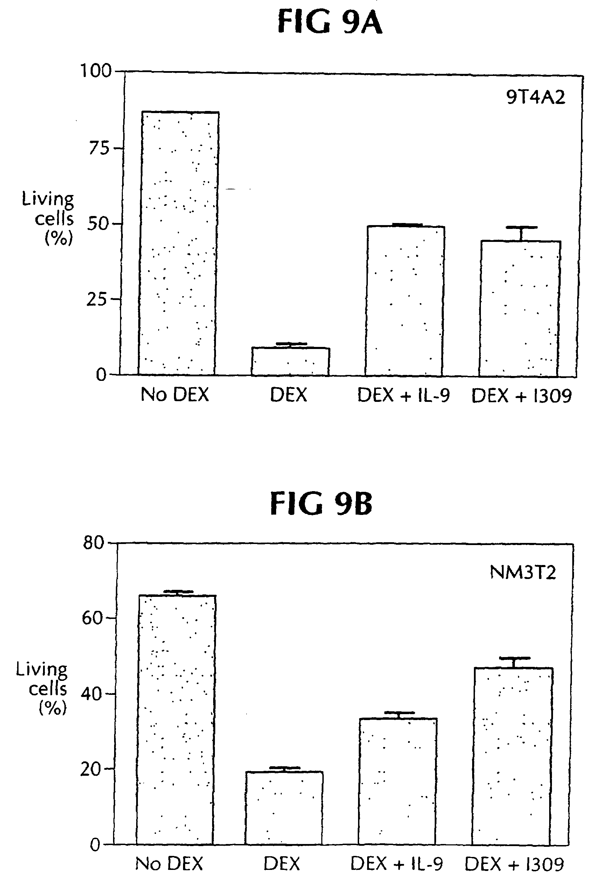

### Anti-apoptotic activity of I-309 for 9T4A2 and NM3T2 thymic lymphoma cell lines exposed to dexamethasone. (Figure 9)

Cells were incubated with dexamethasone in the presence or absence of I-309 (100 U/ml) or IL-9 (500 U/ml) for 20 h. Cell viability was measured by FACS analysis after staining with propidium iodide as described in Material and Methods.

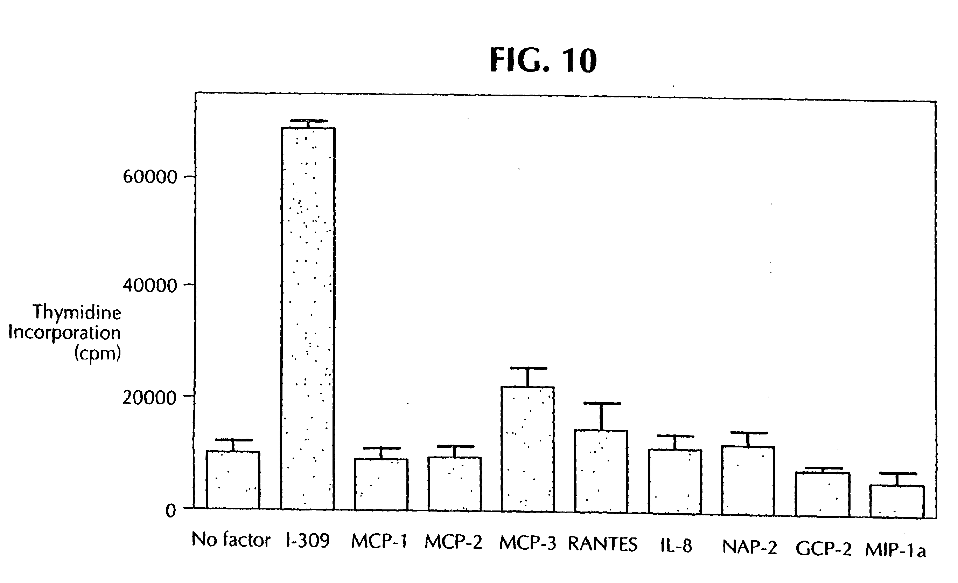

A series of experiments were carried out in which the antiapoptotic activity of various human chemokines on BW5147 was tested. In each case, BW5147 cells (10³/well) were incubated for 3 days in the presence of DEX(0.25 mM), either with or without I-309 (40 ng/ml), MCP-1(25 ng/ml), MCP-2 (250 ng/ml), MCP-3 (250 ng/ml), RANTES (250 ng/ml), IL-8 (125 ng/ml), NAP-2 (125 ng/ml), granulocyte chemotactic protein -2 (250 ng/ml), and macrophage inflammatory protein 1a (125 ng/ml). Cell proliferation was measured in a thymidine incorporation assay, using triplicate cultures, all as described supra. Figure 10 presents these results.

### Results

### Identification of anti-apoptotic activity distinct from IL-6 in human T-cell supernatants

In a previous report, it had been shown that the murine thymic lymphoma BW5147 is protected against dexamethasone-induced apoptosis by IL-9, IL-4 and to a lesser extent by IL-6. By contrast, IL-1, IL-2, IL-7, IL-10, IL-13, IFNγ and TNFα were not active in this assay (Renauld, *et al*, 1995, *Blood* **85**: 1300-1305), as well as IL-3, IL-11, LIF, GM-CSF and Steel Factor (unpublished data). Since human IL-9 and human IL-4 are not active on murine cells, we used this bioassay to screen a series of human supernatants for another anti-apoptotic activity. Since human IL-6 is active on murine cells, we simultaneously measured the IL-6 titers of these supernatants. As shown in Figure 1A, proliferation of BW5147 cells cultured in the presence of dexamethasone was observed with murine IL-9 but also with some human cell supernatants such as those from activated T cell leukaemia (CRL8066-MoT) or from CD4+ T cell clones (5B12, E5). Interestingly, this activity was not strictly correlated with the concentration of IL-6 in these supernatants as shown in Figure 1B. Although the most potent supernatant (activated CRL8066-MoT) contained large amounts of IL-6, other active samples (5B12 and E5 supernatants) were essentially devoid of IL-6, suggesting that a factor distinct from the previously checked cytokines could protect BW5147 cells against dexamethasone-induced apoptosis.

To further discriminate this putative factor from IL-6, CRL8066-MoT cell supernatants, concentrated by adsorption to sulfopropyl sephadex beads, were chromatographed on a size exclusion gel by FPLC. This experiment showed that a factor smaller in size than IL-6 and not inhibited by anti-human IL-6 or anti-mouse IL-6 receptor antibodies was also active in this assay (Figure 2). This conclusion was confirmed by results obtained in a hydrophobic interaction chromatography on phenyl sepharose. As shown in Figure 3, the major anti-apoptotic activity was eluted at 0.75 M ammonium sulphate, while IL-6 was recovered only at 0.0 M salt.

### Purification and micro-sequencing of the factor responsible for the anti-apoptotic activity

CRL8066-MoT cells were produced by stimulating the cells at a concentration of 500,000 cells/ml with PMA at 50 ng/ml for 3 days in medium containing 5% FCS. After chromatography on a Phenyl-Sepharose column, the material was fractionated on a cation exchange gel at pH 5. The active fractions were transferred into a neutral phosphate buffer containing 1 M NACI and a 10⁻⁴ dilution of Tween 20, and subjected to gel filtration chromatography. The anti-apoptotic activity eluted with an apparent molecular weight ranging from 10 to 20 kD with a peak at 15 kD. Further fractionation was carried out by hydrophobic interaction chromatography on a TSK phenyl column from which the active material eluted at 0.7 M Na₂SO₄ and 15% ethylene glycol. Finally, the active fractions were purified to homogeneity on a Resource S cation exchange column by FPLC. The results of this last chromatography step are shown in Figure 4, together with the corresponding SDS PAGE analysis of the active fractions.

The purity of this preparation was confirmed by the fact that HPLC analysis of the most active fraction (fraction 21, see Figure 4) showed a single protein peak, from which the anti-apototic activity was retrieved. Sequencing of 30 pmoles of protein from fraction 21 of the Resource S column (Figure 4) generated the following 27 amino acid sequence: NH₂-Lys-Ser-Met-Gln-Val-Pro-Phe-Ser-Arg-Cys-Cys-Phe-Ser-Phe-Ala-Glu-Gln-Glu-Ile-Pro-Leu-Arg-Ala-Ile-Leu-Cys-Tyr, (SEQ ID NO: 3) which was completely identical to amino acids 1 to 27 of the β-chemokine I-309 (Miller *et al,* 1989, *J*. *Immunol.* **143:** 2907-2916; Miller *et al.*, 1990, *J. Immunol* **145:** 2737-2744).

### Comparison of the specific activity of I-309 in chemotactic and anti-apoptotic in vitro assays.

Based on concentrations deduced from the micro-sequencing analysis, the specific activity of purified I-309 was measured in the anti-apoptotic assay. Maximal protective activity for BW5147 cells was reached at a 3 ng/ml concentration (Figure 5A). If, in this assay, one defines one unit as the concentration required to give half-maximal protection against dexamethasone, one unit would be equivalent to 1.65 ng/ml (approximately 100pM). Interestingly, this specific activity was found to be in a similar range as that of murine IL-4 in the same bioassay (Renauld *et al.*, 1995, *Blood* **85**: 1300-1305).

The same purified material was tested in a chemotactic assay using the human monocytic cell line THP1. As shown in Figure 5B, significant chemotactic responses to I-309 required considerably higher concentrations. Similar results were obtained using normal monocytes (data not shown). Taken together, these data indicate that the anti-apoptotic effect reported here is a much more sensitive assay and that I-309 might be in fact much more effective in vivo as an anti-apoptotic agent than as a chemotactic factor.

### Anti-apoptotic activity of recombinant I-309 and TCA-3.

To formally establish that the anti-apoptotic activity observed with our purified material can be ascribed to I-309 cDNA was amplified by RT-PCR from a PHA activated T cell clone, inserted into an expression vector and transfected into COS-7 cells. As shown in Figure 6, the supernatant from I-309-transfected COS cells strongly stimulated proliferation of BW5147 cells in the presence of dexamethasone, while control COS cell supernatant was totally inactive. In addition, baculovirus derived TCA-3, considered as the murine homologue of I-309, had a similar activity with half-maximal effect observed at approximately 0.5 ng/ml.

### Inhibition of the anti-apoptotic activity of I-309 by Pertussis toxin.

The receptor molecule for I-309/TCA-3 has not yet been identified. However, every chemokine receptor identified so far consist in a transmembrane protein with seven transmembrane domains that transduce the signal through G proteins. Since Pertussis toxin is known to interfere with the activation mediated by a subset of these receptors, we analyzed the effect of this molecule on the anti-apoptotic activity of I-309. As indicated in Figure 8, when BW5147 cells were exposed to dexamethasone in the presence of recombinant I-309, Pertussis toxin completely abrogated the proliferation induced by I-309 in the presence of DEX. By contrast, Pertussis toxin did not inhibit the IL-9-induced cell proliferation, demonstrating that completely distinct mechanisms of signal transduction are involved. These results also suggest that chemokine receptors linked to G proteins may play an unexpected role in the control of cell survival under apoptosis-promoting conditions.

### Anti-apoptotic activity of I-309 on other thymic lymphoma cell lines.

To see whether the anti-apoptotic activity of I-309/TCA-3 can be extended to other thymic lymphoma cell lines, we tested the activity of purified I-309 on two other dexamethasone-sensitive cell lines, 9T4A2 and NM3T2, by measuring cell death 20 hours after exposure to dexamethasone. As shown in Figure 9, I-309 was found to be as potent as IL-9 in inhibiting apoptosis in these cells. These results suggest that the anti-apoptotic activity of I-309 may be a common phenomenon for these types of tumor lines.

The foregoing examples and disclosure describe a method of modulating cell apoptosis. In one aspect of the disclosure the modulation involves the inhibition of cell apoptosis by administering an amount of a β chemokine to a cell sample, in an amount sufficient to inhibit apoptosis of cells in said sample. Especially preferred are proteins which have I-309 activity including mammalian analogous of I-309, such as TCA-3, P500, and so forth, as well as anti-apoptotic fragments of these β chemokines. Especially preferred are methods of inhibiting T cell apoptosis, using the β chemokines in the manner described. "Effective amount" as used herein refers to any amount of the β chemokine sufficient to inhibit cell apoptosis. Preferably, the amount of β chemokine used will range from about 0.1 ng/ml up to about 100 ng/ml. Most preferably, the amount dosed will range from about 1.0 ng/ml to about 3.0 ng/ml. When used in vivo, the protein may be administered in accordance with any of the standard therapeutic regimes, such as orally, intravenously, intramuscularly, subcutaneously, intranasally, or intradermally, with or without a carrier, adjuvant, or additional material to protect the active ingredient from degradation by normal physiological processes. When used in vitro, solutions, emulsion, etc. may be used to facilitate the applications of the material.

One may also inhibit cell apoptosis, as indicated herein, by administering antagonists of β chemokines, such as I-309 antagonists, to a subject. As indicated, these antagonists may be antibodies, such as polyclonal or monoclonal antibodies, soluble forms of the I-309 receptor, and also derivatives of the β chemokines which share sufficient structural similarities with the β chemokines to bind to β chemokine receptors, but sufficiently different so as to be apoptotically inert.

Other aspects. of the invention will be clear to the skilled artisan, and need not be repeated here.

## Claims

1. The use of the β-chemokine I-309 for the manufacture of a medicament for inhibiting heart-attack related apoptosis.

2. The use of the β-chemokine I-309 for the manufacture of a medicament for use in the inhibition of stroke-related apoptosis.

3. The use of the β-chemokine I-309 for the manufacture of a medicament for use in the inhibition of ischaemia related apoptosis.

4. The use of the β-chemokine I-309 for the manufacture of a medicament for use in the inhibition of chemotherapy induced apoptosis.

5. The use for the β-chemokine 1-309 for the manufacture of a medicament for use in the inhibition of radiotherapy induced apoptosis.

6. The use of the β chemokine I-309 for the manufacture if a medicament for use in inhibition of kidney failure related apoptosis.

7. An *in vitro* method for inhibiting apoptosis comprising administering the β chemokine I-309 to a culture of cells.

8. The method of Claim 7 wherein the β chemokine I-309 is administered in an amount ranging from 0.1 ng/ml to 100ng/ml.

9. The method of Claim 8 wherein the β chemokine I-309 is administered in an amount ranging from 1.0 ng/ml to 3.0 ng/ml.

## Patentansprüche

1. Verwendung von β-Chemokin I-309 zur Herstellung eines Medikaments zur Hemmung der Apoptose bei Herzanfall.

2. Verwendung von β-Chemokin I-309 zur Herstellung eines Medikaments zur Hemmung der Apoptose bei Schlaganfall.

3. Verwendung von β-Chemokin I-309 zur Herstellung eines Medikaments zur Hemmung der Apoptose bei Ischämie.

4. Verwendung von β-Chemokin I-309 zur Herstellung eines Medikaments zur Hemmung von Chemotherapie-induzierter Apoptose.

5. Verwendung von β-Chemokin I-309 zur Herstellung eines Medikaments zur Hemmung von Radiotherapie-induzierter Apoptose.

6. Verwendung von β-Chemokin I-309 zur Herstellung eines Medikaments zur Hemmung der Apoptose bei Nierenversagen.

7. *In* vitro-Verfahren zur Apoptosehemmung, umfassend die Verabreichung von β-Chemokin I-309 an eine Zellkultur.

8. Verfahren nach Anspruch 7, wobei das β-Chemokin I-309 in einer Menge im Bereich von 0,1 ng/ml bis 100 ng/ml verabreicht wird.

9. Verfahren nach Anspruch 8, wobei das β-Chemokin I-309 in einer Menge im Bereich von 1,0 ng/ml bis 3,0 ng/ml verabreicht wird.

## Revendications

1. Utilisation de β-chimiokine I-309 pour la fabrication d'un médicament destiné à empêcher une apoptose liée à une crise cardiaque.

2. Utilisation de β-chimiokine I-309 pour la fabrication d'un médicament destiné à l'inhibition d'une apoptose liée à un accident vasculaire.

3. Utilisation de β-chimiokine I-309 pour la fabrication d'un médicament destiné à inhiber l'apoptose liée à une ischémie.

4. Utilisation de β-chimiokine I-309 pour la fabrication d'un médicament destiné à l'inhibition d'une apoptose induite par chimiothérapie.

5. Utilisation de β-chimiokine I-309 pour la fabrication d'un médicament destiné à être utilisé pour l'inhibition d'apoptose induite par radiothérapie.

6. Utilisation de β-chimiokine I-309 pour la fabrication d'un médicament destiné à être utilisé pour l'inhibition d'une apoptose liée à une défaillance de rein.

7. Procédé in vitro pour l'inhibition d'apoptose comprenant l'administration de β-chimiokine I-309 à une culture de cellule.

8. Procédé selon la revendication 7, **caractérisé en ce que** la β-chimiokine I-309 est administrée dans une quantité de 0,1 ng/ml jusqu'à 100 ng/ml.

9. Procédé selon la revendication 8, **caractérisé en ce que** la β-chimiokine I-309 est administrée dans une gamme de 1,0 ng/ml jusqu'à 3,0 ng/ml.
